(19) **Europäisches Patentamt**
**European Patent Office**
**Office européen des brevets**

(11) **EP 3 923 547 A1**

(12) **EUROPEAN PATENT APPLICATION**

(43) Date of publication:
**15.12.2021 Bulletin 2021/50**

(51) Int Cl.:
**H04L 29/08** (2006.01) **A61B 5/00** (2006.01)
**H04L 12/58** (2006.01)

(21) Application number: **20178803.1**

(22) Date of filing: **08.06.2020**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **Medema, Janine Willemijn**
**5656 AE Eindhoven (NL)**
• **Godlieb, Robert**
**5656 AE Eindhoven (NL)**

(74) Representative: **Philips Intellectual Property &
Standards**
**High Tech Campus 52**
**5656 AG Eindhoven (NL)**

(54) **PROVIDING NOTIFICATIONS TO DEVICE USERS**

(57) The invention discloses a computer-implemented notification control method (200) comprising: receiving (202) data relating to the use of at least one device by a user during at least one usage instance; selecting (204) a notification to be presented to the user, the notification having an associated level of pertinence to the user; and determining (206), based on the received data and the level of pertinence of the notification, a time at which to provide the notification for presentation to the user.

Fig. 2

**Description**

FIELD OF THE INVENTION

The invention relates to user notifications and, more particularly, to controlling the provision of notifications to a user based on the manner in which the user uses a device or multiple devices.

BACKGROUND OF THE INVENTION

It is desirable in many technological fields to provide information to a user of a device. For example, it may be intended to present a notification to a user, provide an instruction to a user, request a user to perform an action, and so on. While, in some scenarios, it may be acceptable to provide the information to the user at any time, in some scenarios, it may be desirable to provide certain information or a request for information at particular times. For example, a user may be more receptive to receiving or accepting information, or responding to a request for information, on a particular day of the week, and/or at a particular time of day. At other times when the user is less receptive, the user may not pay sufficient attention to information presented to them, may ignore the information and/or may find the information or request to perform an action irritating or annoying.

One example of a field where information may be presented to a user is the field of personal care, such as monitoring personal health or performing personal care activities. A person may have a personal healthcare regime that includes, for example, washing their face, brushing their teeth and styling their hair each morning, and brushing their teeth each evening before bed. The morning activities may be performed quickly so that the person can leave the house on time, while evening activities may be performed at a more relaxed pace. Based on this example, the user may be more receptive to information during the evening, when they are less rushed than during the morning when they may not have available time to consume the information or act upon the information received.

Many systems may be configured to deliver information (e.g. in the form of notifications) periodically, occasionally or at pre-determined times (e.g. weekly). However, if the predetermined time does not coincide with a time when the user is receptive to information, then the information may not be delivered effectively, or the user may not respond to a requested action.

Therefore, there is a need for a system in which information may be delivered to a user at an appropriate time for the user which coincides with a usage instance of a device, when the user is likely to be more receptive to the information or a requested action, and when the receipt of such information is likely to be more effective or more likely to prompt a response.

SUMMARY OF THE INVENTION

Embodiments disclosed herein seek to address the problem of a notification (e.g. a notification providing information or requesting action to be taken) being delivered to a recipient at an inappropriate time, such as a time when the recipient is unlikely to consume information effectively, or unlikely to perform an action responsive to receiving the notification. The inventors have recognised that data relating to a user's use of particular devices may be used to determine when the user is likely to be more open to having a notification presented to them, so that a determination may be made of when to deliver the notification. For example, it may be determined, based on the user's use of various devices during past usage instances (e.g. previous personal grooming sessions) that use of the devices in a particular way or at a particular time implies that the user is more receptive to receiving information. When a notification is to be delivered to the user, device usage data during a current usage instance may be used to determine whether that usage instance is suitable time at which to deliver the notification.

More specifically, different notifications to be delivered to a user may have different levels of urgency or importance (also referred to as levels of pertinence), and the time at which the information is to be delivered to a user may be based on the pertinence level of the information to be delivered. For example, a notification having a very low level of pertinence (e.g. relatively non-urgent information or a non-urgent request) may be delivered only if the user is determined to be very receptive. Conversely, if the user is determined to be relatively non-receptive, only a notification having a very high level of pertinence (e.g. very important, urgent information or an urgent request) would be delivered to the user.

Various examples are described herein in the context of personal care (e.g. checking blood pressure, tracking heart rate, weight management, hair care, oral care, shaving and the like), but it will be understood that the invention is relevant to many other fields.

According to a first aspect, the invention provides a computer-implemented notification control method comprising: receiving data relating to the use of at least one device by a user during at least one usage instance; selecting a notification to be presented to the user, the notification having an associated level of pertinence to the user; and determining, based on the received data and the level of pertinence of the notification, a time at which to provide the notification for presentation to the user.

In this way, a notification can be provided to the user at an appropriate time, taking into account the user's apparent receptiveness, which can be determined based on various factors relating to how the user uses various devices. By carefully determining an appropriate time to present the notification (e.g. based on data acquired in respect of the user's device use during a current usage instance), the chance of the notification being ignored can be reduced.

In some embodiments, the level of pertinence may vary as a function of time.

The method may, in some embodiments, further comprise determining, based at least on the level of pertinence, a notification threshold that varies as a function of time. In some embodiments, the notification threshold may decrease over time and/or the level of pertinence may increase over time.

In some embodiments, the method may further comprise determining, based on the received data, a level of receptiveness of the user during each of the at least one usage instances to perform an action responsive to receiving the notification.

The method may further comprise responsive to determining, during a usage instance, that the level of receptiveness meets or exceeds the notification threshold for that instance, providing the notification for presentation to the user at that instance.

In some embodiments, the method may further comprise, responsive to determining that the notification threshold has decreased to zero without a usage instance occurring where the level of receptiveness meets or exceeds the notification threshold, providing the notification for presentation to the user.

The notification to be presented may, in some embodiments, comprise an instruction for the user to perform an action. The method may further comprise, responsive to determining that, within a defined duration, the user has not performed the action, determining a further time at which to provide the notification for presentation to the user.

In some embodiments, the received data may comprise an indication of at least one of: a manner in which the user has used the device, a day on which the user uses the device, a time of day at which the user uses the device, an amount of time spent by the user using the device, a number of activities performed using the device, movements made by the user, facial expressions of the user, emotions of the user, ambient conditions during the at least one usage instance, and a number of other users within a defined distance of the user during the at least one usage instance.

According to a second aspect, the invention provides a computer program product comprising a non-transitory computer-readable medium, the computer-readable medium having computer-readable code embodied therein, the computer-readable code being configured such that, on execution by a suitable computer or processor, the computer or processor is caused to perform the method of any of the preceding claims.

According to a third aspect, the invention provides a notification control apparatus comprising a processor configured to: receive data relating to the use of at least one device by a user during at least one usage instance; select a notification to be presented to the user, the notification having an associated level of pertinence to the user; and determine, based on the received data and the level of pertinence of the notification, a time at which to provide the notification for presentation to the user.

In some embodiments, the at least one device may comprise a personal health device selected from a group comprising: a shaving device, a hair cutting device, a hair styling device, a skin care device, a skin cleansing device, a skin hydration sensing device, a skin treatment device, an oral care device, an interactive mirror, a sleep tracking device, a health monitoring device, an activity monitoring device, an intense pulsed light hair removal device, a thermometer and a weighing scale.

The apparatus may further comprise a presentation module configured to present a notification to a user.

According to a fourth aspect, the invention provides a notification device comprising a notification control apparatus as disclosed herein. The notification device may comprise a device selected from a group comprising: a laptop computer, a tablet computer, a smartphone, a smart speaker, a wearable computing device and a smart mirror.

According to a fifth aspect, the invention provides a personal care device comprising a notification control apparatus as disclosed herein.

These and other aspects of the invention will be apparent from and elucidated with reference to the embodiments described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 is an illustration of an example of a bathroom setting;
Fig. 2 is a flowchart of an example of a notification control method;
Fig. 3 shows two plots of example data generated according to embodiments of the invention;
Fig. 4 is an enlarged portion of one of the plots shown in Fig. 3;

Fig. 5 is a flowchart of a further example of a notification control method;

Fig. 6 is a schematic illustration of a machine-readable medium in communication with a processor;

Fig. 7 is schematic illustration of an example of a notification control apparatus;

Fig. 8 is a schematic illustration of a further example of a notification control apparatus;

Fig. 9 is a schematic illustration of an example of a notification device; and

Fig. 10 is a schematic illustration of an example of a personal care device.

DETAILED DESCRIPTION OF THE EMBODIMENTS

Embodiments disclosed herein provide a mechanism for determining an appropriate time for delivering information to a person, based on analysis of data relating to the user's use of one or more devices. In the context of this invention, the term "device" is intended to cover any type of device which can be monitored during use, for example using a sensor in the device itself or a sensor external to the device. The disclosed embodiments are applicable to a wide range of fields. In some examples described herein, the invention is described in the context of personal care, with some examples involving the analysis of data relating to a user's use of one or more personal care devices including, for example, a bathroom scale, a power toothbrush, or a shaving device.

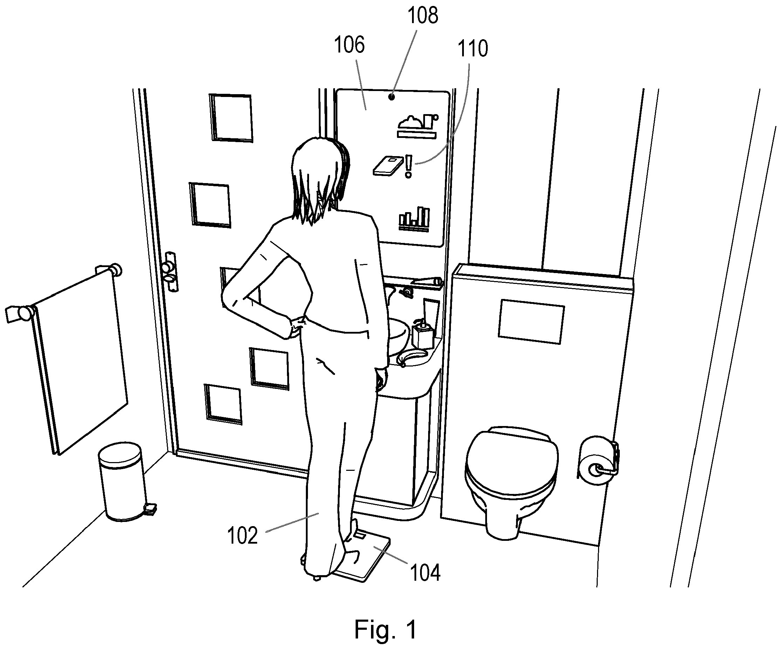

An example of a setting in which embodiments of the present disclosure may be used is shown in Fig. 1. Fig. 1 is an illustration of a bathroom setting. A user 102 may visit a bathroom to perform personal care activities, such as brushing their teeth, styling their hair, and the like. In the example shown in Fig. 1, the user 102 is standing on a bathroom scale 104 in order to measure their weight. More generally, however, the user 102 may be using any device 104. In the example shown in Fig. 1, the bathroom also includes an interactive mirror 106. An interactive mirror, sometimes referred to as a smart mirror, is a unit which, in addition to functioning as a mirror to show a user his or her reflection, is also capable of displaying information to the user. Information, such as text, images and videos, may be displayed on a display portion of the interactive mirror which may, for example, be positioned behind a mirrored (or partially mirrored) panel or a mirrored (or partially mirrored) surface. In this way, the display screen, or portions thereof, may be visible through the mirror portion, so that a user is able to simultaneously view their reflection and information presented on the display screen. The interactive mirror 106 may include one or more sensors, such as an imaging device 108 (e.g. a camera) capable of capturing an image (e.g. of the user 102) which can be displayed on the screen of the interactive mirror. The camera 108 may also be used to detect the presence of a person standing in front of, or approaching, the interactive mirror 106 so that the interactive mirror can be powered on or "woken up" when the presence of a person is detected, to detect the identity of a user of the mirror, to detect activity of the user and/or to measure ambient conditions of or around a user. The interactive mirror 106 may be connected (wirelessly or via a wired connection) to one or more other devices including the bathroom scale 104. The processing circuitry within the interactive mirror 106 may receive data from the bathroom scale 104 (and/or from other connected devices) and may display information to the user relating to the connected device and/or relating to measurements acquired using the connected device. In other examples, other devices may be used to present information to a user, such as a smartphone, a smart speaker, a tablet computer, and the like.

The user 102 may, in some examples, be following a health management program and, as part of the programme, it may be intended that the user measures and records their weight periodically (e.g. daily or weekly) or at intervals determined by the health management program. In some examples, the interactive mirror 106 may be configured to recognise when a weight measurement has not been recorded on a particular occasion, or for a defined period of time, and may display a notification 110 on the display of the interactive mirror. For example, the notification 110 may remind the user 102 to take regular measurements of their weight or to request the user to measure their weight immediately.

Even though information (e.g. the notification 110) may be displayed on the screen of the interactive mirror 106, the user 102 may on some occasions pay little or no attention to the information. For example, if the user is rushing to perform a number of personal care activities in the morning before leaving their house, then they may not have sufficient time to fully consume or take on board the information presented to them. In the example above, if the user 102 ignores the notification 110 reminding them to record their weight, then the interactive mirror 106 may continue to present the notification to the user constantly or periodically (e.g. each time the user is detected to be using the interactive mirror) until the user adheres to the request and records their weight. The provision of constant or regular notifications on the interactive mirror 106 may be perceived by the user 102 as annoying, and this may have a negative effect on the user, discouraging them from performing the requested action (i.e. measuring their weight). According to embodiments of the present invention, therefore, data from one or more devices 104 (e.g. the bathroom scale and/or other personal care/healthcare devices) is used to determine when the user 102 is most likely to be receptive to information, such as requests to perform a particular action.

The receptiveness of a person (e.g. the user 102) to information may vary widely from person to person and from time to time, depending on a number of factors such as their lifestyle, their personal care regime, and the emotion or mood of the user. For example, a person who is in a rush to perform personal care activities quickly (e.g. in a bathroom in the morning before work on a day the person overslept) may not be very receptive to information presented to them. In contrast, however, a person performing a personal care activity in the evening may not have as strict restrictions regarding their time, so may be more receptive to information or a request to perform a particular task or action. Data relating to the use of one or more devices including, for example, data indicative of a day and/or time of the use of the device may be used to determine when a user appears to be more receptive to information and, in some cases, may be used to recognise patterns in a user's personal care regime. More specifically, such data may be used to determine an appropriate time when the user is most likely to be more receptive to the information being presented. According to embodiments, it is possible to recognise from historic data from past device usage instances when the user is most receptive, and a notification can be presented to the user when data acquired in respect of the user's use of one or more devices during a current usage instance implies that a similar level of receptiveness reached during that usage instance.

In addition to the receptiveness of the user, an indication of the importance of the information to be presented may be used to determine when the information is to be presented. For example, it may be determined appropriate to present information considered to be of particular importance or urgency (e.g. having a high level of pertinence) to the user at a particular time even though the user is less receptive, because it may be considered important that the user receives the information more urgently, even if there is a chance that the user may not fully absorb the information, or may find it more irksome to perform a task in response to a request. As is apparent from the discussion below, the trade-off between the level of pertinence of the information and the receptiveness of the user may vary over time, for example as the intended or optimum window of time for presenting the information to the user runs out.

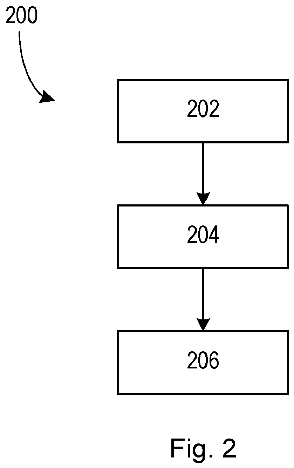

According to a first aspect, a method is provided. Fig. 2 is a flowchart of an example of a method 200, which may be considered to be a notification control method. The method 200 may be referred to as a computer-implemented method as it may be performed using processing apparatus, such as one or more processors. The method 200 comprises, at step 202, receiving data relating to the use of at least one device 104 by a user 102 during at least one usage instance. A usage instance may involve the use of one or more than one device during the same session. For example, a usage instance may comprise a toothbrushing session performed by a user, or multiple activities, such as toothbrushing and shaving. The data may be acquired using one or more sensors in the device 104 (or each device) itself (e.g. an accelerometer, a timer, a force sensor, or the like) or one or more sensors external to the device, capable of acquiring information in relation to the use of the device (e.g. a camera, a microphone, or the like).

At step 204, the method 200 comprises selecting a notification to be presented to the user, the notification having an associated level of pertinence to the user 102. The notification may comprise any type of information of which the user is to be notified. In some examples, the notification may be selected from a plurality of predefined (e.g. preprepared) notifications, which may be stored in a storage medium (e.g. a memory), for example in a database accessible by a processor performing the method 200. In some examples, each notification may be stored with an indication of an associated level of pertinence to the user. The level of pertinence may vary over time and/or between users. In other words, a level of pertinence for a notification is personal for a particular user. For example, the pertinence of a notification relating to a health management program may vary over the course of the health management program. The pertinence of such a notification may increase as the user progresses through the program, and/or as the time approaches a nominal/optimal time for presenting the notification to the user. For each notification, there may be an optimum time of presentation to the user. However, as noted above, presentation of the notification may not be effective if the user is not receptive to the information at that time.

The method 200 comprises, at step 206, determining, based on the received data and the level of pertinence of the notification, a time at which to provide the notification for presentation to the user 102. The determination of step 206 may be considered to be a determination of a particular moment during a usage instance (e.g. a personal care session being performed by the user) at which to provide the notification. In a basic scenario, for example, data from a power toothbrush may be recorded each time a user 102 uses the toothbrush to brush their teeth which may, for example, be twice daily. The received data may indicate that, on some occasions, the user brushes their teeth for less than a recommended 2 minutes and, on some days, skips a brushing session altogether. Therefore, the method 200 may select (at step 204) a notification advising the user 102 to brush their teeth for 2 minutes and, at step 206, the method 200 may determine that the notification should be provided for presentation to the user 102 as soon as the user picks up the toothbrush for the next brushing session. If data from the toothbrush indicates that the user continues to brush their teeth for less than the recommended 2 minutes, then the level of pertinence of the notification may increase, as it becomes more important/urgent that the user adheres to the advice presented in the notification. If the user fails to brush their teeth for two days in a row, a different notification may be selected at step 204, such as a notification advising of the importance of brushing teeth for oral health and full body health. This notification may not focus on brushing for 2 minutes, but may instead focus on brushing teeth in general and, due to the importance of this advice, the notification may be presented to the user when the user is determined (e.g. from device usage data) to be at all receptive to the information. Once it is determined that the user is brushing their teeth regularly, then the notification relating to 2 minutes of brushing may again be selected, but this will be presented when the device usage data indicates that the user's receptiveness level is high.

In these examples, data relating to use of the device is received and used in determining the time or moment during a usage instance or session at which the notification should be presented. The received data may comprise an indication of at least one of: a manner in which the user has used the device, a day on which the user uses the device, a time of day at which the user uses the device, an amount of time spent by the user using the device, a number of activities performed using the device, movements made by the user, facial expressions of the user, emotions of the user, ambient conditions during the at least one usage instance and a number of other users within a defined distance of the user during the at least one usage instance. Combinations of various types of data may be used to determine or decide whether or not to present the notification and, if so, the time or moment at which to present the notification. Data indicating the day on which the user uses the device may be recorded along with data indicating the time of day at which the user uses the device. Data indicating an amount of time spent by the user using each device during each usage instance may provide understanding of how much time is available to the user to perform a particular activity with the device. This may be used to determine whether the user is in a relaxed state/mood or appearing rushed during a usage instance, and this can be indicative of the receptiveness of the user. When considered alone or in combination with other data, the data indicating the amount of time spent using a device may provide understanding of usage instances when the user spends the most time performing activities using a particular device, and when the user appears to be rushed while using the device. Data indicating a number of activities performed using the device may also provide an insight into how relaxed a user is during a usage instance, indicative of the receptiveness of the user. For example, if the user 102 uses a particular device (e.g. an interactive mirror) to perform multiple personal care activities during a usage instance, then it may be determined that the user has more time available during that particular usage instance. Data indicating a manner in which the user has used a device may provide an understanding of an amount of time available to the user. Such data may be acquired using one or more sensors of the device or associated with the device, and the data may indicate that the user is using the device as intended or, conversely, incorrectly, in an apparently rushed manner. Data indicating movements made by the user may be used to interpret whether or not the user is in a rush. Data indicating facial expressions of the user may be used to determine a mood of the user and, therefore, the user's susceptibility to taking on board information during that particular usage instance. Data indicating ambient conditions during the at least one usage instance may be used, for example, to determine light levels in the vicinity of the user and/or whether or not any additional people are present. Data may also indicate a number of other users within a defined distance of the user during the at least one usage instance. Such data may give clues as to whether the user appears relaxed and whether information is likely to be received well. For example, if an additional person is present during a usage instance, then the user is likely to be less receptive, since they may spend some of their time focussing on the other person. Furthermore, some notifications may be of a personal or private nature.

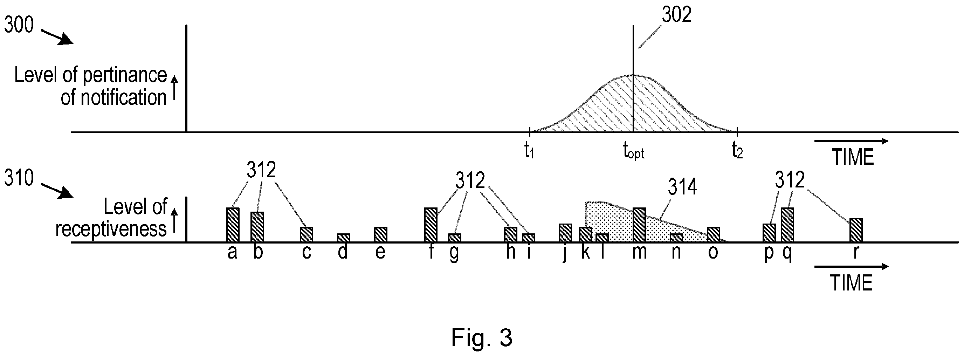

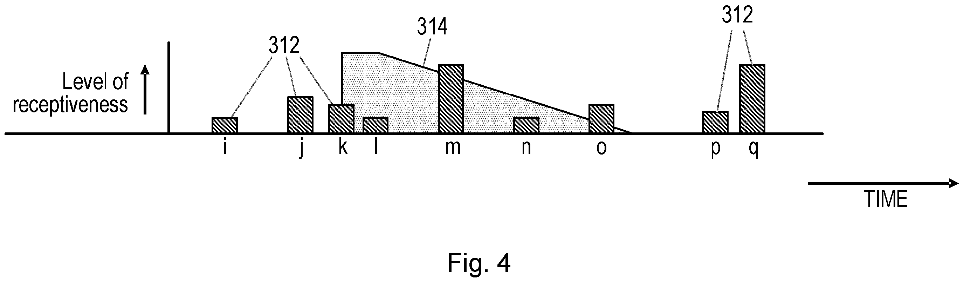

More detailed examples of embodiments will now be described with reference to Figs. 3 and 4, which show plots of data acquired and used according to an example of the invention. Fig. 3 shows a first plot 300 showing how the level of pertinence of a notification varies as a function of time according to one example. In this example, an intended optimum notification time $t_{opt}$ is indicated at 302 and, at this time, the pertinence level of the notification is at a maximum. The optimum notification time or moment is the time or moment at which the notification should ideally be presented to the user 102. The optimum notification time may, for example, comprise a time/moment relative to the usage instances of the user. For example, the optimum notification time may be the start of the user's next usage instance (e.g. as soon as the user picks up a shaving device). In some examples, the level of pertinence may remain constant over time while, in other examples, (including the example shown in Fig. 3) the level of pertinence may vary as a function of time. In the example of Fig. 3, the level of pertinence of the notification varies approximately according to a bell curve or Gaussian curve centred on the optimum notification time 302. Thus, in this example, the level of pertinence increases according to the Gaussian function from 0 at a time $t_1$ to the maximum $t_{opt}$ 302, and decreases according to the Gaussian function from the maximum 302 to 0 at a time $t_2$. Such a function may be used in relation to a notification whose relevance is time-limited. For other notifications, different functions may be applied, such as a function that ramps up to a maximum pertinence level.

Fig. 3 also shows a second plot 310 of various device usage instances 312a to 312r over time. Each usage instance 312 is represented as a bar having a height indicative of a level of receptiveness of the user during the usage instance. The level of receptiveness of the user 102 during a particular instance of usage of a device may be determined, as discussed above, based on the received data relating to the use of the device by the user. In the example shown, the usage instances 312a, b, f, m and q have levels of receptiveness that are significantly higher than the receptiveness levels of other usage instances in the plot 310. The relatively high receptiveness levels of the user during the usage instances 312a, b, f, m and q may, for example, correspond to particular days and/or times when the user appears, from the received data, to be taking part in a more relaxed usage instance, so likely has more time available and, therefore, is more likely to fully consume and take on board any information provided in a notification at that time.

Data acquired relating to the use of the at least one device may, in some embodiments, be stored (e.g. in a storage facility, such as a memory) and used to determine a level of receptiveness of the user during each usage instance. The stored data (sometimes referred to as historic data) may also be used to detect patterns or trends that might indicate periodic usage instances during which the user 102 is particularly receptive to information. The historic data may also be used to determine a baseline against which to compare any newly-acquired data. Thus, previously-acquired data is used as a reference for newly-acquired data.

According to some examples, determining a time at which to provide the notification for presentation to the user 102 may comprise simply detecting a usage instance (e.g. detecting that the user is using a device) during the period of time that the notification has a non-zero level of pertinence. For example, based on the example shown in Fig. 3, the period of time between $t_1$ to $t_2$ may be determined as an appropriate time to present the notification, if a usage instance occurs during that time window. In other examples, however, a more appropriate time may be determined. For example, it may be determined that the notification will be presented to the user 102 if the level of pertinence of the notification and the level of receptiveness of the user are sufficiently high. One way of determining the time according to various embodiments is to determine a notification threshold based on at least the level of pertinence of the notification. The notification threshold may vary as a function of time. In some examples, both the level of pertinence and the notification threshold may vary over time. As a usage instance takes place (e.g. the user begins to use a device), then the level of receptiveness of the user can be determined based on data acquired from or in respect of the device. If, for a particular usage instance, the level of receptiveness of the user exceeds the notification threshold, then it may be determined that the receptiveness of the user at that time is high enough for the notification to have an intended effect and, therefore, the notification may be presented to the user at that time. In some examples, the notification may be provided for presentation to the user as soon as the determined level of receptiveness of the user reaches the notification threshold.

In some embodiments, the notification threshold may decrease over time, meaning that, as time passes, it becomes more acceptable to present the notification to the user 102 even though the level of receptiveness of the user may be relatively lower. This scenario represents the increase in need to present the notification to the user 102 while it is still relevant, even if the user may not be at their most receptive. In other words, it may be considered that patience is running out, and that the notification is to be presented to the use regardless of the user's receptiveness.

The plot 310 of Fig. 3 shows an example of a notification threshold varying as a function of time, based on the level of pertinence of the notification shown in the plot 300. An enlarged portion of the plot 310 is shown in Fig. 4, for improved clarity. Specifically, Fig. 4 shows a portion of the plot 310 that includes the time during which the usage instances 312i to 312q occur. In some examples, the notification threshold 314 may decrease over time. In the example shown, the notification threshold 314 is constant for a portion of time, before decreasing at steady rate over time to 0. The constant portion of the notification threshold begins at a time (corresponding to the usage instance 312k. The notification threshold is a threshold that must be met by the user's level of receptiveness during a particular usage instance in order for the notification to be provided for presentation to the user 102. In the example shown in Fig. 4, data received during the usage instances 312k and 312l indicated that, during those usage instances, the user 102 had a relatively low level of receptiveness, which fell short of the notification threshold 314. During the usage instance 312m, data relating to the use of the device(s) by the user indicates that the user's level of receptiveness increases to beyond the notification threshold 314 during the usage instance. Responsive to determining during a particular usage instance that the level of receptiveness meets or exceeds the notification threshold at that instance, the notification may be provided for presentation to the user. Thus, based on the acquired usage data, it is determined that the user 102 is likely to be receptive to the notification, and the notification may be provided with the expectation or hope that the user will absorb and act upon the information provided.

In some embodiments, if the notification threshold decreases to 0 before a usage instance takes place during which the level of receptiveness of the user meets or exceeds the notification threshold, then the notification may be provided for presentation to the user at the next possible opportunity, such as the next detected usage instance. This ensures that the notification is presented to the user even if the user's level of receptiveness does not meet the intended threshold.

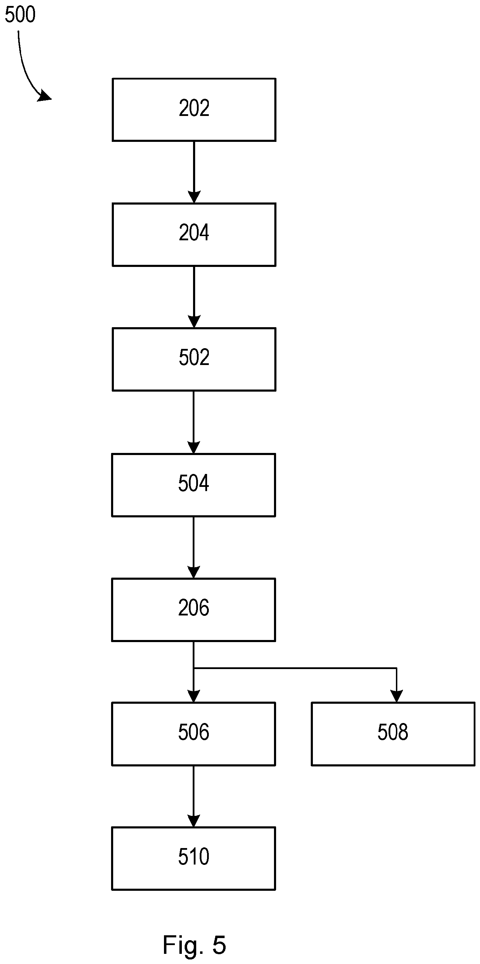

Fig. 5 is a flowchart of a further example of a notification control method 500. The method 500, which may comprise a computer-implemented method, may comprise one or more steps of the method 200 discussed above. The method 500 may comprise, at step 502, determining, based at least on the level of pertinence, a notification threshold that varies as a function of time. In other examples, the notification threshold may remain constant, for example if the presentation of the notification is not time-constrained. As noted above, the notification threshold may, in some examples, decrease over time. Alternatively or additionally, the level of pertinence of the notification may increase over time. In this way, it may be considered that it is of increasing importance that the notification is presented to the user, and the likelihood that the notification will be presented to the user therefore increases over time.

At step 504, the method 500 may comprise determining, based on the received data, a level of receptiveness of the user during each of the at least one usage instances to perform an action responsive to receiving the notification. The action to be performed may, in some examples, comprise simply reading the notification while, in other examples, the action to be performed may be more involved, such as taking a measurement, entering some data, completing a questionnaire, watching a video, and the like. Thus, in some examples, the level of receptiveness may also be based on the nature of the action to be performed by the user once the notification has been presented. For example, the received data may imply that the user is receptive to simply reading a notification, but not to watching a video.

The level of receptiveness of a user may be determined in a number of ways, and may take into account varying amounts of data, depending for example on the amount of data available. According to one example, the level of receptiveness, $R$, may be determined using the following function:

$$R = \frac{(8n + 2Tn + 5M + T + D)}{x} (n - nh + Tn - Tnh + M - Mh + T - Th + D - Dh)$$

where R is the level of receptiveness, T is the current time of day, Th is the historical time of day, D is the current day of the week, Dh is the historical day of week, n is the number of devices used during the current usage instance, nh is the historical number of devices used during each usage instance, Tn is the current device usage duration, Tnh is the historical device duration, M is the amount of physical movement of current device, Mh is the historical physical movement of the device, and x is a compensating factor to ensure R is in a range between 0 and 100. The historical values may comprise an average of values measured during multiple previous usage instances. The values 5, 8 and 2 in the function represent weighting factors that are specific to a user, and these may vary from user to user. The weighting factors may be calculated or selected (e.g. automatically or manually).

The first factor in the function (i.e. $\frac{(T+5D+8n+2Tn+M)}{x}$) represents data for the current usage instance, while the second factor in the function (i.e. $(T - Th + D - Dh + n - nh + Tn - Tnh + M - Mh)$) represents the difference between the current usage instance and historical use (i.e. data received during past usage instances). It will be appreciated that the above function represents just one example of a way of determining a level of receptiveness, and alternative functions or techniques may be used.

The method 500 may comprise, at step 506, responsive to determining, during a usage instance, that the level of receptiveness meets or exceeds the notification threshold for that instance, providing the notification for presentation to the user at that instance. From the above discussion, it will be understood that the level of receptiveness may increase as a usage instance progresses. For example, as the user 102 spends longer performing a personal care activity and/or uses more devices during the usage instance, it may be determined that the user is relaxed and, therefore, more receptive to information being presented to them. Once the level of receptiveness increases to the notification threshold (or beyond the notification threshold), the notification may be provided for presentation. For example, a processor performing the method may transmit a signal instructing a presentation unit to present the notification. Alternatively, the processor may cause the notification to be presented itself.

Once the notification has been provided for presentation to the user, the task to present the notification to the user may be considered complete, and the method 200, 500 may begin again in respect of a different notification. In some examples, however, if the notification comprises a request for user action, a further step of receiving a user response (e.g. an indication that the user has performed the requested action) may be required for the method to be considered complete.

In some examples, the user 102 may not take part in a usage instance (e.g. the user might not perform a personal care activity) during the period of relevance for the notification (e.g. between $t_1$ and $t_2$ in Fig. 3). Thus, in examples where the notification threshold decreases to 0, a usage instance may not occur at all, or a usage instance may not occur where the level of receptiveness of the user exceeds the notification threshold. In this example, a decision may be taken to present the notification to the user (e.g. during the next usage instance, such as the next time the user is detected to be present at the interactive mirror) even though the level of receptiveness of the user is not considered to be high enough. Thus, at step 508, the method 500 comprises, responsive to determining that the notification threshold has decreased to zero without a usage instance occurring where the level of receptiveness meets or exceeds the notification threshold, providing the notification for presentation to the user.

As discussed above, the notification may, in some examples, simply provide information to the user which the user is expected to read while, in other examples, the notification may comprise a request or instruction for the user to perform a particular action. If it is determined that the user performs the requested action following the provision of the notification, then the method 200, 500 may end and/or may begin again with a different notification. If, however, the user does not perform the requested action, then a time may be determined at which to present the notification again. For example, if a notification containing a request for an action is ignored by the user, the notification will remain in a list of notifications to be delivered, and the level of pertinence may be adapted in time. On the next occasion that the notification threshold is reached, the notification will be again presented to the user. Thus, in some examples, the notification to be presented may comprise an instruction for the user to perform an action. In such examples, the method 500 may comprise, at step 510, responsive to determining that, within a defined duration, the user has not performed the action, determining a further time at which to provide the notification for presentation to the user.

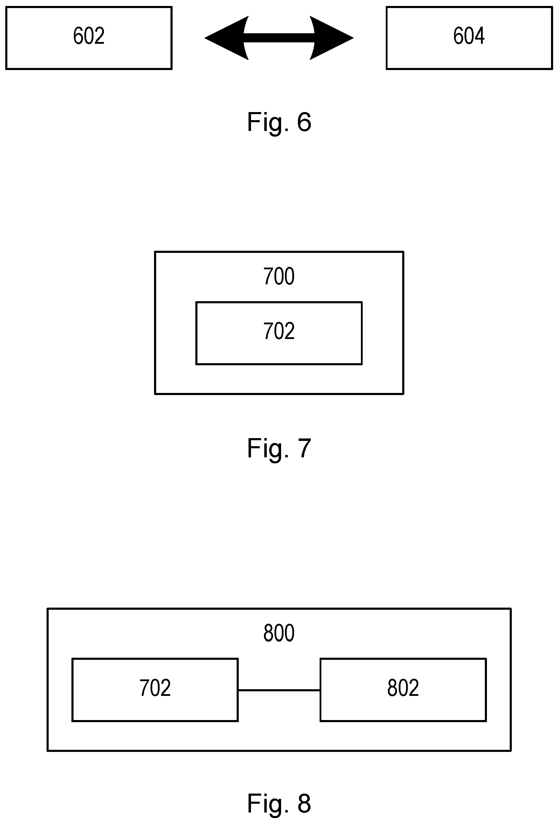

According to a second aspect, a computer program product is provided. Fig. 6 is a schematic illustration of an example of a processor 602 in communication with a computer-readable medium 604. According to various embodiments, a computer program product comprises a non-transitory computer-readable medium 604, the computer-readable medium having computer-readable code embodied therein, the computer-readable code being configured such that, on execution by a suitable computer or processor 602, the computer or processor is caused to perform steps of the methods 200, 500 discussed herein. Thus, the methods 200, 500 may be performed using one or more processors or processing apparatuses.

According to a third aspect, an apparatus is provided. Fig. 7 is a schematic illustration of an example of a notification control apparatus 700. The apparatus 700 comprises a processor 702, which may be configured to perform steps of the methods 200, 500 discussed herein. According to some embodiments, the processor 702 is configured to receive data relating to the use of at least one device by a user during at least one usage instance; select a notification to be presented to the user, the notification having an associated level of pertinence to the user; and determine, based on the received data and the level of pertinence of the notification, a time at which to provide the notification for presentation to the user.

The at least one device from which data is received may comprise any type of device capable of being monitored, for example using one or more sensors of the device itself or one or more external sensors. In some embodiments, the at least one device may comprise a personal health device or a personal care device. The device may be a device selected from a group comprising a shaving device, a hair cutting device, a hair styling device, a skin care device, a skin cleansing device, a skin hydration sensing device, a skin treatment device, an oral care device, an interactive mirror, a sleep tracking device, a health monitoring device, an activity monitoring device, an intense pulsed light hair removal device, a thermometer and a weighing scale. In other embodiments, another type of device may be used.

Fig. 8 is a schematic illustration of a further example of a notification control apparatus 800. The apparatus 800 comprises the processor 702 and may further comprise a presentation module 802 configured to present a notification to the user. For example, the presentation module 802 may receive the notification, or a signal instructing the presentation of the notification, from processor 702 when the notification is to be presented. For example, the presentation module 802 may receive the signal or notification when it is determined that the level of receptiveness of the user during a particular usage instance meets or exceeds the notification threshold at that instance. The presentation module 802 may comprise any module or unit capable of presenting the notification to the user. For example, presentation module 802 may include one or more of a display screen, a speaker, a light or LED, a tactile delivery unit and an olfactory delivery unit.

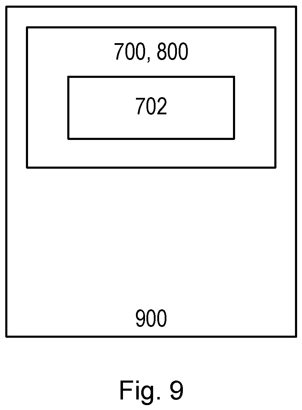

According to a fourth aspect, a notification device is provided. Fig. 9 is a schematic illustration of an example of a notification device 900. The notification device comprises a notification control apparatus 700, 800 as disclosed herein. The notification device 900 may be a device selected from a group comprising a laptop computer, a tablet computer, a smartphone, a smart speaker, a wearable computing device and a smart mirror. Thus, the notification device may receive data relating to the use of the one or more devices being used by the user (e.g. personal care devices), perform the steps of the methods 200, 500, and present the notification to the user at the appropriate time. In this example, therefore, the notification control apparatus may be embodied in a computing device. In other examples, the notification control apparatus 700, 800 may be embodied in a personal care device itself.

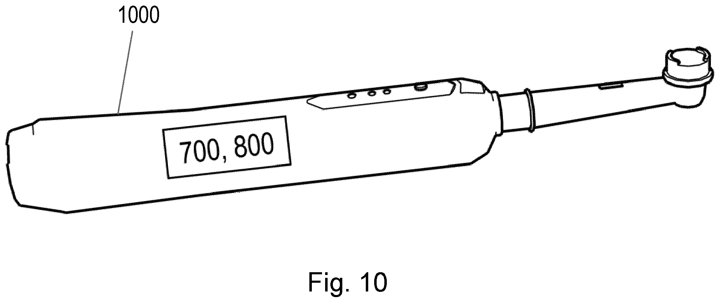

According to a fifth aspect, a personal care device is provided. Fig. 10 is a schematic illustration of an example of a personal care device 1000. In the example shown in Fig. 10, the personal care device 1000 comprises a power toothbrush. However, it will be appreciated that the personal care device may comprise any personal health or care device, such as those discussed herein. The personal care device 1000 comprises a notification control apparatus 700, 800 as discussed herein. Thus, the methods 200, 500 discussed herein may be performed by the personal care device 1000 while a user is using it. For example, one or more sensors of the personal care device 1000 may acquire data relating to its use, and the processor of the notification control apparatus in the personal care device 1000 may process the data and determine a time at which to deliver the notification to the user. The notification may, in some embodiments, be presented to the user via a presentation module (e.g. a user interface) of the device itself.

It will be appreciated that various modifications may be made to the examples discussed herein, without departing from the scope of the claims. For example, the notification control apparatus 700, 800, the notification device 900 and/or the personal care device 1000 may comprise a storage unit (e.g. a memory) configured to store code to be executed by a processor and/or configured to store historic data (i.e. data relating to the use of the one or more devices during past usage instances). Such data may be stored in or in associated with a user profile, such that each user's usage data may be recalled and used in the method when the user is detected to be taking part in a usage instance. In other embodiments, such data may be stored remotely, such as in a server.

Embodiments disclosed herein provide a mechanism by which a determination may be made of an appropriate time or moment during a usage instance at which to provide a notification for presentation to a user of a device, based on acquired data relating to the user's use of devices. Based on data acquired during past usage instances, and data acquired during a current usage instance, a determination can be made regarding whether or not the current usage instance constitutes an appropriate time for the notification to be presented. More specifically, the determination is made based on a level of receptiveness of the user, which may be determined based on the received data relating to the usage of the device or devices. By making such a determination, notifications may be presented to the user at a time when the user is most likely to accept and consume the notification and, if required, take action in response to the notification. This can improve the user experience while using devices, because the user may not become frustrated or annoyed as a result of continuous appearances of notifications.

The processor 602, 702 can comprise one or more processors, processing units, multi-core processors or modules that are configured or programmed to control the apparatus 700, 800 in the manner described herein. In particular implementations, the processor 602, 702 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, FLASH storage or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented notification control method (200, 500) comprising:

   receiving (202) data relating to the use of at least one device by a user during at least one usage instance;
   selecting (204) a notification to be presented to the user, the notification having an associated level of pertinence to the user; and
   determining (206), based on the received data and the level of pertinence of the notification, a time at which to provide the notification for presentation to the user.

2. A method (200, 500) according to claim 1, wherein the level of pertinence varies as a function of time.

3. A method (200, 500) according to claim 1 or claim 2, further comprising:
determining (502), based at least on the level of pertinence, a notification threshold that varies as a function of time.

4. A method (200, 500) according to claim 3, wherein the notification threshold decreases over time and/or the level of pertinence increases over time.

5. A method (200, 500) according to claim 3 or claim 4, further comprising:
determining (504), based on the received data, a level of receptiveness of the user during each of the at least one usage instances to perform an action responsive to receiving the notification.

6. A method (200, 500) according to claim 5, further comprising:
responsive to determining, during a usage instance, that the level of receptiveness meets or exceeds the notification threshold for that instance, providing (506) the notification for presentation to the user at that instance.

7. A method (200, 500) according to claim 5 or claim 6, further comprising:
responsive to determining that the notification threshold has decreased to zero without a usage instance occurring where the level of receptiveness meets or exceeds the notification threshold, providing (508) the notification for presentation to the user.

8. A method (200, 500) according to any of the preceding claims, wherein the notification to be presented comprises an instruction for the user to perform an action; and
wherein the method further comprises:
responsive to determining that, within a defined duration, the user has not performed the action, determining (510) a further time at which to provide the notification for presentation to the user.

9. A method (200, 500) according to any of the preceding claims, wherein the received data comprises an indication of at least one of: a day on which the user uses the device, a time of day at which the user uses the device, an amount of time spent by the user using the device, a number of activities performed using the device, a manner in which the user has used the device, movements made by the user, facial expressions of the user, emotions of the user, ambient conditions during the at least one usage instance, and a number of other users within a defined distance of the user during the at least one usage instance.

10. A computer program product comprising a non-transitory computer-readable medium (604), the computer-readable medium having computer-readable code embodied therein, the computer-readable code being configured such that, on execution by a suitable computer or processor, the computer or processor (602) is caused to perform the method of any of the preceding claims.

11. A notification control apparatus (700) comprising:
a processor (702) configured to:

   receive data relating to the use of at least one device by a user during at least one usage instance;
   select a notification to be presented to the user, the notification having an associated level of pertinence to the user; and
   determine, based on the received data and the level of pertinence of the notification, a time at which to provide the notification for presentation to the user.

12. An apparatus (700) according to claim 11, wherein the at least one device comprises a personal health device selected from a group comprising: a shaving device, a hair cutting device, a hair styling device, a skin care device, a skin cleansing device, a skin hydration sensing device, a skin treatment device, an oral care device, an interactive mirror, a sleep tracking device, a health monitoring device, an activity monitoring device, an intense pulsed light hair removal device, a thermometer and a weighing scale.

13. An apparatus (700, 800) according to claim 11 or claim 12, further comprising:
a presentation module (802) configured to present a notification to a user.

**14.** A notification device (900) comprising:

a notification control apparatus (700, 800) according to any of claims 11 to 13;
wherein the notification device comprises a device selected from a group comprising: a laptop computer, a tablet computer, a smartphone, a smart speaker, a wearable computing device and a smart mirror.

**15.** A personal care device (1000) comprising:
a notification control apparatus (700, 800) according to any of claims 11 to 13.

Fig. 1

200

202

204

206

Fig. 2

Fig. 3

Fig. 4

500

```
      ┌──────────────┐
      │     202      │
      └──────────────┘
             │
             ▼
      ┌──────────────┐
      │     204      │
      └──────────────┘
             │
             ▼
      ┌──────────────┐
      │     502      │
      └──────────────┘
             │
             ▼
      ┌──────────────┐
      │     504      │
      └──────────────┘
             │
             ▼
      ┌──────────────┐
      │     206      │
      └──────────────┘
             │
             ├───────────────────────┐
             ▼                        ▼
      ┌──────────────┐         ┌──────────────┐
      │     506      │         │     508      │
      └──────────────┘         └──────────────┘
             │
             ▼
      ┌──────────────┐
      │     510      │
      └──────────────┘
```

Fig. 5

602 ⟷ 604

Fig. 6

700

702

Fig. 7

800

702 — 802

Fig. 8

700, 800

702

900

Fig. 9

Fig. 10

1000

700, 800

Europäisches Patentamt

European Patent Office

Office européen des brevets

## EUROPEAN SEARCH REPORT

Application Number

EP 20 17 8803

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 10 051 107 B1 (PRASAD RICHA [US] ET AL) 14 August 2018 (2018-08-14) | 1,2, 5-11,13 | INV. H04L29/08 A61B5/00 H04L12/58 |
| Y | * column 1, line 5 - column 17, line 30; figures 1-9 * | 12,14,15 | |
| X | EP 3 273 395 A1 (FUJITSU LTD [JP]) 24 January 2018 (2018-01-24) * paragraph [0002] - paragraph [0064]; figures 1-5 * | 1,2, 5-11,13 | |
| X | US 2016/248865 A1 (DOTAN-COHEN DIKLA [IL] ET AL) 25 August 2016 (2016-08-25) * paragraph [0016] - paragraph [0144]; figures 2-6 * | 1,2, 4-11,13 | |
| X | US 2012/295645 A1 (YARIV ERAN [IL] ET AL) 22 November 2012 (2012-11-22) * paragraph [0001] - paragraph [0047]; figures 1-6 * | 1-3,8, 10,11,13 | |
| Y | WO 2017/032547 A1 (KONINKLIJKE PHILIPS NV [NL]) 2 March 2017 (2017-03-02) * page 1, line 3 - page 13, line 8 * | 12,14,15 | TECHNICAL FIELDS SEARCHED (IPC) H04L A61B |

The present search report has been drawn up for all claims

| Place of search | Date of completion of the search | Examiner |
|---|---|---|
| Munich | 18 November 2020 | Lupia, Sergio |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 17 8803

This annex lists the patent family members relating to the patent documents cited in the above-mentioned European search report.
The members are as contained in the European Patent Office EDP file on
The European Patent Office is in no way liable for these particulars which are merely given for the purpose of information.

18-11-2020

| Patent document cited in search report | | Publication date | Patent family member(s) | | Publication date |
|---|---|---|---|---|---|
| US 10051107 | B1 | 14-08-2018 | AU | 2018234490 A1 | 22-08-2019 |
| | | | BR | 112019016572 A2 | 31-03-2020 |
| | | | CA | 3053987 A1 | 20-09-2018 |
| | | | CL | 2019002559 A1 | 07-02-2020 |
| | | | CN | 110419206 A | 05-11-2019 |
| | | | CO | 2019009580 A2 | 18-09-2019 |
| | | | EP | 3566425 A1 | 13-11-2019 |
| | | | JP | 2020516110 A | 28-05-2020 |
| | | | KR | 20190128165 A | 15-11-2019 |
| | | | PH | 12019550177 A1 | 08-06-2020 |
| | | | SG | 11201908067V A | 27-09-2019 |
| | | | US | 10051107 B1 | 14-08-2018 |
| | | | US | 2018343338 A1 | 29-11-2018 |
| | | | WO | 2018169763 A1 | 20-09-2018 |
| | | | ZA | 201905229 B | 28-10-2020 |
| EP 3273395 | A1 | 24-01-2018 | CN | 107644313 A | 30-01-2018 |
| | | | EP | 3273395 A1 | 24-01-2018 |
| | | | JP | 2018014087 A | 25-01-2018 |
| | | | US | 2018026920 A1 | 25-01-2018 |
| US 2016248865 | A1 | 25-08-2016 | CN | 107251065 A | 13-10-2017 |
| | | | EP | 3259720 A1 | 27-12-2017 |
| | | | US | 2016248865 A1 | 25-08-2016 |
| | | | US | 2019356750 A1 | 21-11-2019 |
| | | | WO | 2016133700 A1 | 25-08-2016 |
| US 2012295645 | A1 | 22-11-2012 | NONE | | |
| WO 2017032547 | A1 | 02-03-2017 | BR | 112018003372 A2 | 25-09-2018 |
| | | | CN | 107921650 A | 17-04-2018 |
| | | | EP | 3341167 A1 | 04-07-2018 |
| | | | JP | 2018525124 A | 06-09-2018 |
| | | | RU | 2018110091 A | 26-09-2019 |
| | | | US | 2018236675 A1 | 23-08-2018 |
| | | | WO | 2017032547 A1 | 02-03-2017 |

EPO FORM P0459

For more details about this annex : see Official Journal of the European Patent Office, No. 12/82